# EUROPEAN PATENT SPECIFICATION

(11) **EP 4 450 016 B1**
(45) Date of publication and mention of the grant of the patent: **02.09.2026**
(21) Application number: 22921816.9
(22) Date of filing: 18.01.2022
(51) Int. Cl.: A61B 34/37, A61B 18/12, A61B 34/30, A61B 34/00, A61B 18/00

(54) **OPERATION TOOL**
OPERATIONSWERKZEUG
OUTIL D'ACTIONNEMENT

(43) Date of publication of application: 23.10.2024
(73) Proprietor: RIVERFIELD Inc., Tokyo 107-0052 (JP)
(72) Inventor: SHINDO, Koki, Tokyo 107-0052 (JP)
(74) Representative: Adares PartGmbB
(86) International application number: PCT/JP2022/001630
(87) International publication number: WO 2023/139654

(56) References cited:
- WO-A1-2012/043463
- JP-A- 2015 525 614
- JP-A- 2018 505 001
- JP-B1- 6 747 745
- US-A1- 2006 079 889
- US-A1- 2011 313 405
- US-A1- 2018 228 562
- US-A1- 2018 325 579

## Description

### TECHNICAL FIELD

The present disclosure relates to a surgical instrument.

### BACKGROUND ART

In regard to a master-slave surgical robot, as a method of driving a surgical instrument mounted on such surgical robot, various methods have been proposed. For example, as disclosed in Patent Document 1, there is a known method of driving the surgical instrument using a driving force generated by a driving source such as an actuator and transmitted through a transmitter or a wire.

Patent Document 2 describes a surgical instrument comprising a force transmission housing which can be made of PEEK, a roll drive input shaft which can be made of PEEK and roll into the housing, a roll drive input capstan on the roll drive input shaft, an instrument shaft, a roll drive output capstan on the instrument shaft, a roll drive tendon routed around the roll drive input capstan and around the roll drive output capstan, an electrical connector; and an electrical conductor. A first end of the electrical conductor is electrically coupled to the electrical connector. The electrical conductor is routed around the roll drive input capstan and around the roll drive output capstan. A second end of the electrical conductor is electrically coupled to the instrument shaft.

Patent Document 3 describes surgical instrument comprising an end effector, a control link, an electrical conductor, electrical contacts, and an actuator comprising a rotatable spool for receiving a length of the electrical conductor wound around the spool and connecting to the electrical contacts.

Patent Document 4 describes a robotic electrosurgical tool including an end effector cartridge, an elongated shaft, and a tool base. The tool base comprises an electrical connector coupled to an electrosurgical generator, a spool, a drum and cable loops. The tool base can be enclosed by a cover which mounts the electrical connector for the conductor to permit connection to the electrosurgical generator. Further, the robotic electrosurgical tool comprises an insulated conductor passing out of the shaft to the electrical connector for connecting to the electrical connector.

Patent Document 5 describes a tool apparatus. The tool apparatus includes an actuator housing, and an elongate tool manipulator extending outwardly from the actuator housing and having a plurality of control links extending along a length of the tool manipulator. The apparatus also includes a plurality of actuators, each actuator being associated with at least one of the control links and being mounted in the actuator housing to facilitate a range of travel in a transverse direction substantially orthogonal to the actuating direction, and a plurality of linkages.

### PRIOR ART DOCUMENTS

### PATENT DOCUMENTS

Patent Document 1: Japanese Patent No. 6747745
Patent Document 2: US 2011/313405 A1
Patent Document 3: US 2018/325579 A1
Patent Document 4: US 2006/079889 A1
Patent Document 5: US 2018/228562 A1

### SUMMARY OF THE INVENTION

### PROBLEMS TO BE SOLVED BY THE INVENTION

Examples of the surgical instrument to be mounted on the surgical robot are an electric scalpel, forceps, and the like that use high-frequency current. In such surgical instrument using the high-frequency current, a conductor that supplies the high-frequency current to the forceps and the like is provided inside the surgical instrument. Meanwhile, the wires used to transmit the driving force may be made of metallic materials such as stainless steel so as to satisfy required conditions such as strength and flexibility.

In a case that the wires are made of conductive metallic materials, the wires need to be insulated to inhibit the high-frequency current from flowing to other portions than an intended objective portion. For example, a certain distance needs to be set to ensure the insulation between the wires and the conductor. Such a distance, which is required to ensure the insulation between the wires and the conductor, is hereinafter also referred to as a "spatial distance".

The wires may be housed in a cartridge so as to be relatively movable with respect to the cartridge. In such cases, it is necessary to make a distance between a position of the wires closest to the conductor within their moving ranges and a position of the conductor wider than the spatial distance.

By doing so, the cartridge of the surgical instrument, in which the wires and the conductor are housed, becomes larger. When the cartridge becomes larger, in a case that a plurality of surgical instruments is mounted on the surgical robot, distances between each position of the surgical instruments also become wider.

When using the surgical robot equipped with such surgical instruments to perform surgery such as robotic endoscopic surgery, it is necessary to widen distances between a plurality of ports. A port is a member to be placed at a site of surgery and is a tubular member into which a shaft of the surgical instrument is inserted.

If the distances between the plurality of ports are wider, various limitations tend to occur when trying to approach an objective tissue to be operated by using the surgical instruments mounted on the surgical robot. This has raised an issue of increase in clinical cases that are difficult to apply to the surgical robot.

The present disclosure discloses one example of the surgical instruments which more easily inhibits the distances between each of their positions from being widened when mounted on the surgical robot.

### MEANS FOR SOLVING THE PROBLEMS

The problem is solved according to a surgical instrument according to claim 1. The surgical instrument as one aspect of the present disclosure comprises at least one transmitter arranged to be relatively movable with respect to a
casing, at least one wire having at least one portion held in the at least one transmitter, the at least one wire transmitting movement of the at least one transmitter to a movable part, a conductor arranged in the casing, the conductor conducting an electric power that is externally supplied to the movable part, an insulator having insulating capacity, the insulator covering an end portion of the at least one wire extending from the at least one transmitter to a side other than that of the movable part, the insulator comprising a covering area with the at least one wire arranged inside the insulator and a non-covering area with none of the at least one wire arranged inside the insulator.

The at least one transmitter of the surgical instrument as one aspect of the present disclosure is arranged to be relatively movable in a linear manner with respect to the casing.

According to the surgical instrument with this configuration, the end portions of the wires extending to a side other than that of the movable part are covered by the insulator. The insulator includes the non-covering area with none of the wires arranged inside the insulator, thus making it easier to maintain the insulation even without increasing a distance between the end portions of the wires and the conductor. In other words, this configuration makes it easier to reduce a size of the casing in which the wires and the conductor are housed.

The non-covering area of the insulator of the surgical instrument as one aspect of the present disclosure has a specified length greater than or equal to a spatial distance that is determined based on the electric power to be supplied to the conductor.

According to the surgical instrument with this configuration, the non-covering area has the specified length. The specified length is greater than or equal to a spatial distance that is determined based on the electric power to be supplied to the conductor, so that it is easier to maintain the insulation between the end portions of the wires and the conductor.

The insulator of the surgical instrument as one aspect of the present disclosure covers a circumference of a plurality of end portions of a plurality of wires.

According to the surgical instrument with this configuration, the plurality of end portions of the plurality of wires are brought together and covered by the insulator. By covering the plurality of end portions of the plurality of wires with the insulator together, it is easier to maintain the insulation between such end portions and the conductor.

### EFFECTS OF THE INVENTION

A surgical instrument of the present disclosure makes it easier to ensure insulation between end portions of wires and a conductor even without increasing a distance therebetween, and to reduce a size of a casing in which the wires, a conductor, and the like are housed. Accordingly, the surgical instruments of the present disclosure, when mounted on the surgical robot, produce an effect of easily inhibiting distances between each of their positions from being widened.

### BRIEF DESCRIPTION OF THE DRAWINGS

FIG. 1 is a perspective view explaining an external view of a surgical instrument according to the present disclosure.
FIG. 2 is a perspective view explaining an external view of the surgical instrument according to the present disclosure.
FIG. 3 is a perspective view explaining a configuration of a first casing.
FIG. 4 is a perspective view explaining a position relationship between a second casing and, wires and sliders.
FIG. 5 is a perspective view explaining a position relationship between the second casing and the wires.
FIG. 6 is a partial enlarged view explaining a configuration of an insulator.
FIG. 7 is a perspective view explaining a configuration of the wires and a slider.

### EXPLANATION OF REFERENCE NUMERALS

1...surgical instrument, 20...casing, 26...wire, 27...end portion, 21A...conductor, 40...slider (transmitter), 60...insulator, 61...covering area, 62...non-covering area

### MODE FOR CARRYING OUT THE INVENTION

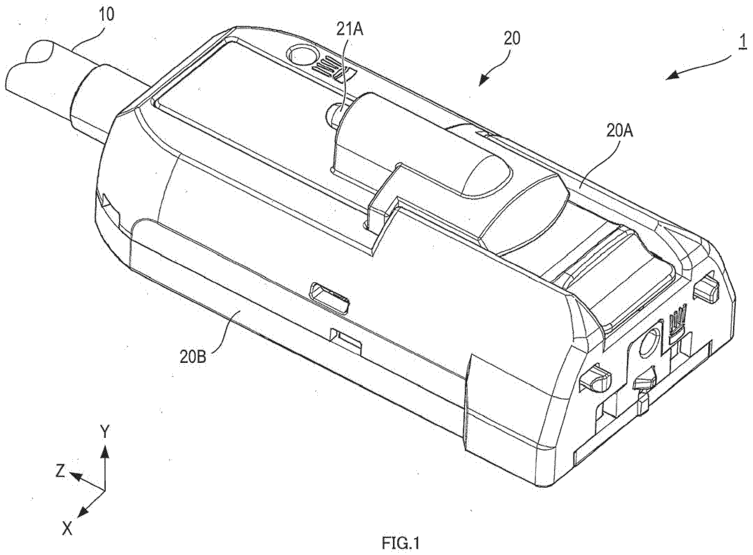

A surgical instrument according to an embodiment of the present disclosure is described with reference to FIGS. 1 through 7. A surgical instrument 1 according to the present embodiment is an instrument to be provided on a multi-degree-of-freedom manipulator in a surgical robot that is remotely controllable. The surgical instrument 1 may have a configuration of a forceps or the like for use in treating a patient during endoscopic surgery, for example.

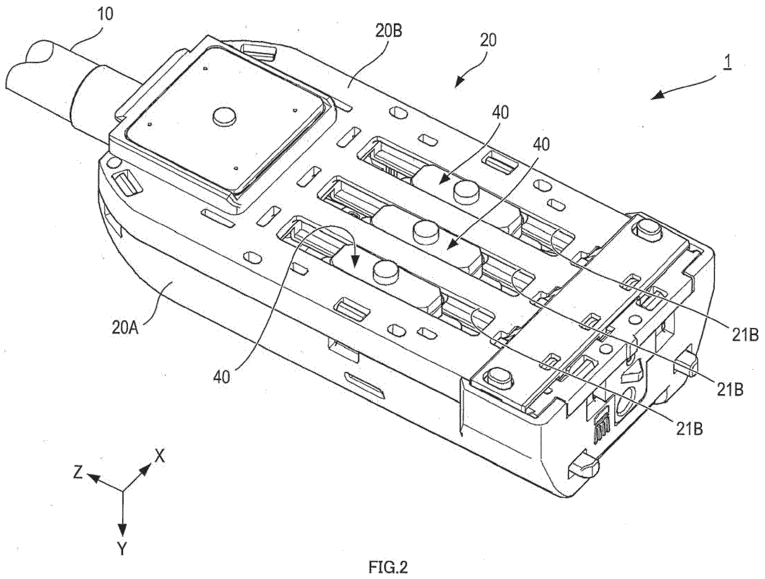

As shown in FIGS. 1 and 2, the surgical instrument 1 is provided with a shaft 10 and a casing 20.

To simplify the description of the present embodiment, a direction in which the shaft 10 extends is described as a Z-axis, and a direction toward a leading end from a base of the shaft 10 is described as a positive direction of the Z-axis. In addition, a direction which is orthogonal to the Z-axis and along which later-described two or more sliders 40 (corresponding to transmitters) are aligned is described as an X-axis, and a left direction when facing in the positive direction of the Z-axis is described as a positive direction of the X-axis. Further, a direction orthogonal to the Z-axis and to the X-axis is described as a Y-axis, and a direction from the surface where the sliders 40 are aligned in the casing 20 to an opposite surface is described as a positive direction of the Y-axis.

The shaft 10 is a rod-shaped member to be inserted into a body of a patient. The shaft 10 is disposed to extend from the casing 20 in the positive direction of the Z-axis. The shaft 10 is configured to have a columnar or cylindrical shape.

For example, the shaft 10 is provided with a joint and forceps (not shown) at its leading end in the positive direction of the Z-axis. The joint and the forceps correspond to movable parts.

The joint is configured to allow changes in orientations of the forceps using the driving force transmitted from the sliders 40 (described below). A specific configuration of the joint may be a general configuration that allows changes in orientations of forceps using a driving force transmitted, and is not particularly limited to any specific configuration.

The forceps have a configuration similar to general forceps for treatment of a patient. In the present embodiment, as an example, a case in which the forceps are arranged at the leading end of the shaft 10 is described, but other instruments to be used for treatment of a patient may be arranged at the leading end of the shaft 10.

The casing 20 comprises a first casing 20A and a second casing 20B. The first casing 20A and the second casing 20B are members forming an outer shape of the casing 20.

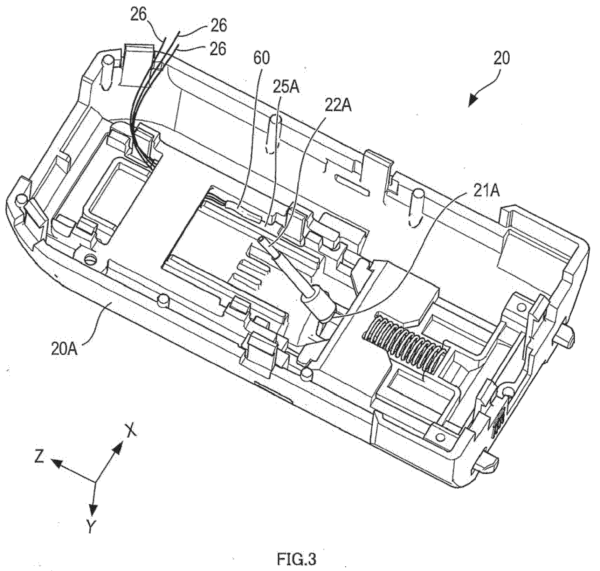

As shown in FIGS. 1 and 3, the first casing 20A of the casing 20 is a member forming side faces and a top face of the casing 20, which faces are portions on a positive side in the Y-axis direction. The first casing 20A comprises a conductor 21A. The conductor 21A is a member that is arranged on the top face of the first casing 20A and that conducts externally-supplied high-frequency current to the forceps.

The conductor 21A is made of a conductive material, such as a metal material. The conductor 21A of the present embodiment is made of copper or an alloy containing copper as an ingredient. The conductor 21A is arranged through the first casing 20A and is electrically connected inside the casing 20 to a conductive wire 22A that conducts the high-frequency current to the forceps.

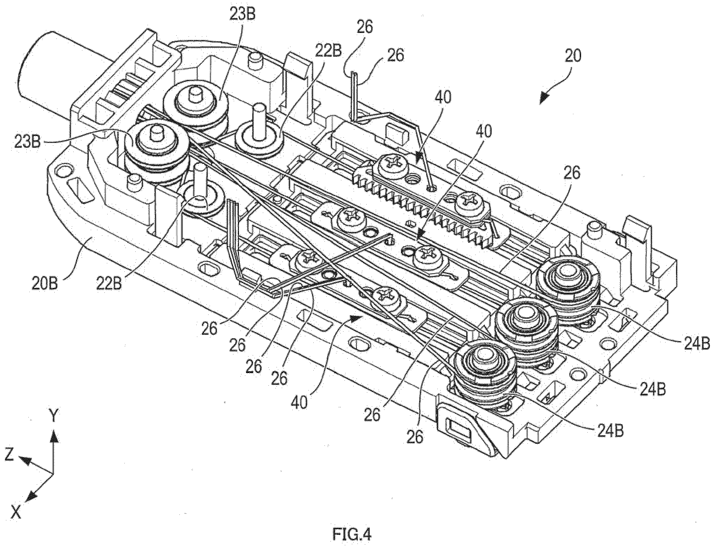

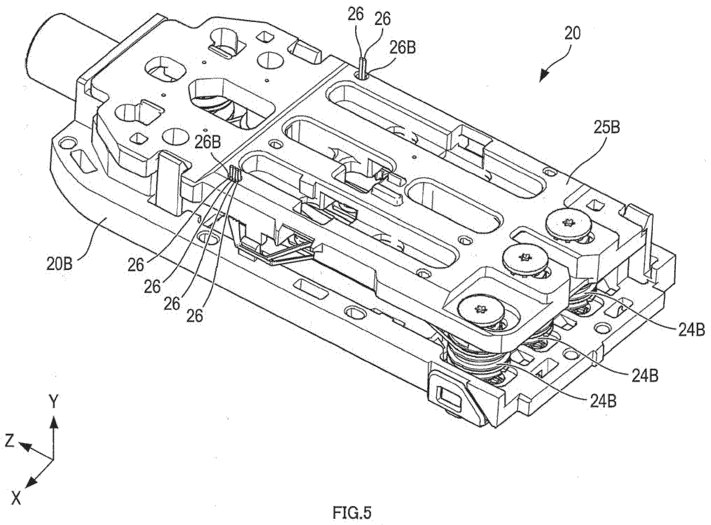

As shown in FIGS. 1 and 2, the second casing 20B is a plate-shaped member forming a bottom face of the casing 20 on a negative side in the Y-axis direction. As shown in FIG. 2, the second casing 20B is provided with three driven grooves 21B and three sliders 40. Further, as shown in FIG. 4, the second casing 20B is provided with three wires 26. Furthermore, as shown in FIG. 5, the second casing 20B is provided with a cover 25B.

As shown in FIG. 2, the driven grooves 21B are elongated holes provided in an end face of the second casing 20B located on a negative side in the Y-axis direction. In other words, the driven grooves 21B are elongated holes provided on an attachment/detachment face where the second housing 20B and the multi-degree-of-freedom manipulator are attached/detached. Further, the driven grooves 21B are configured to extend along the Z-axis.

The three driven grooves 21B are arranged side by side at equal intervals in the X-axis direction. The number of the driven grooves 21B may be determined based on motions or the like of the joint, the forceps and/or the like. In other words, the number of the driven grooves 21B may be determined based on motions in accordance with a specification required for the multi-degree-of-freedom manipulator. The number of the driven grooves 21B may be more than three or less than three, in accordance with the specification required.

The sliders 40 are configured to receive the driving force from the multi-degree-of-freedom manipulator, and then transmit the driving force to the joint and/or forceps. The sliders 40 are configured to be attachable to and detachable from the multi-degree-of-freedom manipulator.

The sliders 40 are arranged to be relatively movable with respect to the casing 20. The present embodiment is described as applied to a case in which the sliders 40 are arranged to be relatively movable in a linear manner with respect to the casing 20. The sliders 40 may be arranged to be relatively rotatable with respect to the casing 20.

One slider 40 is arranged on each of the three driven grooves 21B so as to be movable within the driven groove 21B in the Z-axis direction. In other words, the sliders 40 are arranged so as to be relatively movable in a linear manner with respect to the casing 20. The slider 40 may be arranged on each of all driven grooves 21B, or on only some of the driven grooves 21B.

Some of the three sliders 40 may be configured to transmit the driving force to the forceps. The rest of the sliders 40 is/are configured to transmit the driving force to the joint. For example, two of the three sliders 40 are configured to transmit the driving force to the forceps. Out of the three sliders 40, one slider 40, other than the sliders 40 transmitting the driving force to the forceps, is configured to transmit the driving force to the joint.

The wires 26 shown in FIG. 4 are configured to transmit the driving force from the sliders 40 to the forceps or the joint. One slider is provided with one or two wires 26.

The wires 26 are wires each formed in a long shape using a conductive material. In the present embodiment, as an example, a case is described in which the wires 26 are made of metal materials such as stainless steel, tungsten, an alloy containing tungsten as an ingredient, and a piano wire (e.g., as specified in JIS G 3522), which are used in manipulators in surgical robot systems.

The cover 25B shown in FIG. 5 is a plate-shaped member mounted on the second casing 20B and is a member arranged on the positive side in the Y-axis direction with respect to the sliders 40. The cover 25B includes wire holes 26B through which the wires 26 are inserted.

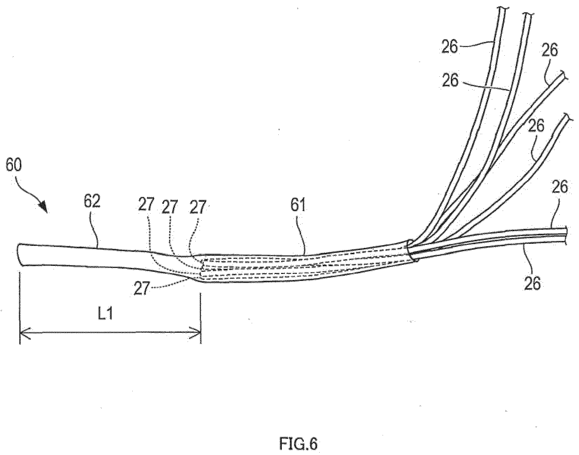

As shown in FIG. 6, an insulator 60 is provided on end portions 27 of the wires 26 that extend from the sliders 40 to a side other than that of the forceps or the joint. More specifically, the insulator 60 is provided on the end portions 27 of the wires 26 that are inserted through a later-described insertion hole 46 of the slider 40.

As shown in FIG. 5, the wires 26 are inserted through the wire holes 26B of the cover 25B from the slider 40. The insulator 60 is arranged on the end portions 27 of the wires 26 guided from the wire holes 26B to the positive-side in the Y-axis direction.

The insulator 60 is a member covering a circumference of a plurality of end portions 27 of a plurality of wires 26. The present embodiment is described using, as an example, a case in which one insulator 60 covers the end portions 27 of all the wires 26. However, one insulator 60 may cover an end portion 27 of one wire 26.

The insulator 60 is a tubular member made of an insulating material. For example, the insulator 60 is made of an insulating resin material. The insulator 60 is preferably made of a resin material that will shrink when heat is applied.

As shown in FIG. 6, the insulator 60 includes a covering area 61 and a non-covering area 62 that are continuously arranged in a longitudinal direction of the insulator 60. The covering area 61 is an area where the wires 26 are arranged inside the insulator 60 in an inner part of a cross-section that intersects with the longitudinal direction of the insulator 60, in other words, inside a tubular shape. The non-covering area 62 is an area where the wires 26 are not arranged inside the insulator 60.

The non-covering area 62 has a specified length L1. The specified length L1 has a length greater than or equal to a distance required to ensure electrical insulation, which is determined based on voltage of the high-frequency current supplied to the conductor 21A, for example. Examples of the necessary distance may include a spatial distance.

A numerical value of the specified length L1 is preferably in the range of 3 mm or more and 80 mm or less, and more preferably in the range of 3 mm or more and 40 mm or less. According to the voltage of the high-frequency current supplied to the conductor 21A or the like, the numerical value of the specified length L1 is selected from the aforementioned range.

The high-frequency current supplied to the conductor 21A is used for electrocautery during surgery, for example. By setting the numerical value of the specified length L1 to 3 mm or more, a minimum required spatial distance can be secured. By setting the numerical value of the specified length L1 to 80 mm or less, it is easier to inhibit reduction of workability, and by setting the numerical value of the specified length L1 to 40 mm or less, it is much easier to inhibit reduction of workability.

Reduction of workability includes reduction of workability to combine the first casing 20A and the second casing 20B to each other. If the specified length L1 becomes longer, it is more difficult to arrange the wires 26 and the insulator 60 in a placement portion 25A, and something such as the wires 26 is likely get caught between the first casing 20A and the second casing 20B. Accordingly, it is necessary to work with care to ensure that wires do not get caught, which will be liable to reduction of workability.

As shown in FIG. 3, the wires 26, the end portions 27 of which are covered by the insulator 60, are inserted into the groove-shaped placement portion 25A, which is arranged on an inner surface of the first casing 20A, from the positive side of the Z-axis direction to a negative side of the Z-axis direction. In FIG. 3, to make the figure easier to see, only three of six wires 26 are shown. The placement portion 25A is a portion formed to extend in the Z-axis directions. The conductor 21A is provided at a position adjacent to the placement portion 25 on the negative side in the Z-axis direction in the first casing 20A.

As shown in FIG. 4, two first guide pulleys 22B that guide the wires 26 to the shaft 10, two second guide pulleys 23B, and three third guide pulleys 24B are provided within the second casing 20B of the casing 20.

The first guide pulleys 22B are arranged closer to the shaft 10 than the sliders 40 are. The two first guide pulleys 22B are arranged side by side, one of which is on a positive side in the X-axis direction, and the other is on a negative side in the X-axis direction.

The two first guide pulleys 22B each guide, to the two second guide pulleys 23B, the wire 26 extending from the slider 40 located on the positive side in the X-axis direction and the wire 26 extending from the slider 40 located on the negative side in the X-axis direction.

The second guide pulleys 23B are arranged closer to the shaft 10 than the sliders 40 are, similarly to the first guide pulley 22B. The two second guide pulleys 23B are arranged side by side, one of which is on the positive side in the X-axis direction, and the other is on the negative side in the X-axis direction.

The two second guide pulleys 23B guide, to an inside of the shaft 10, the wire 26 extending from the first guide pulley 22B located on the positive side in the X-axis direction and the wire 26 extending from the first guide pulley 22B located on the negative side in the X-axis direction.

The third guide pulleys 24B are arranged more away from the shaft 10 than the sliders 40 are. The three third guide pulleys 24B are arranged side by side in the X-axis direction. Each of the third guide pulleys 24B is formed in a cylindrical shape and includes two or more grooves on its cylindrical surface so as to guide the corresponding wire 26.

In the present embodiment, as an example, a case is described in which three grooves are provided at different positions in the Y-axis direction on the cylindrical surfaces of each third guide pulley 24B. Each of the three third guide pulleys 24B is configured to allow the wire 26 to move from one groove that guides the wire 26 to a groove adjacent thereto.

Out of the three third guide pulleys 24B, the third guide pulley 24B on the negative side in the X-axis direction causes the wire 26 extending from the slider 40 located on the negative side in the X-axis direction to move from the groove that is first guiding the wire 26 to an adjacent groove, so that the wire 26 is differently positioned in the Y-axis direction and then guided to the second guide pulley 23B located on the negative side in the X-axis direction. The second guide pulley 23B on the negative side in the X-axis direction guides such wire 26 to the inside of the shaft 10.

The third guide pulley 24B in the center causes the wire 26 extending from the slider 40 in the center to move from the groove that is first guiding the wire 26 to an adjacent groove, so that the wire 26 is differently positioned in the Y-axis direction and then guided to the second guide pulley 23B located on the positive side in the X-axis direction. The second guide pulley 23B on the positive side in the X-axis direction guides the wire 26 to the inside of the shaft 10.

The third guide pulley 24B on the positive side in the X-axis direction causes the wire 26 extending from the slider 40 located on the positive side in the X-axis direction to move from the groove that is first guiding the wire 26 to an adjacent groove, so that the wire 26 is differently positioned in the Y-axis direction and then guided to the second guide pulley 23B located on the positive side in the X-axis direction. The second guide pulley 23B on the positive side in the X-axis direction guides the wire 26 to the inside of the shaft 10.

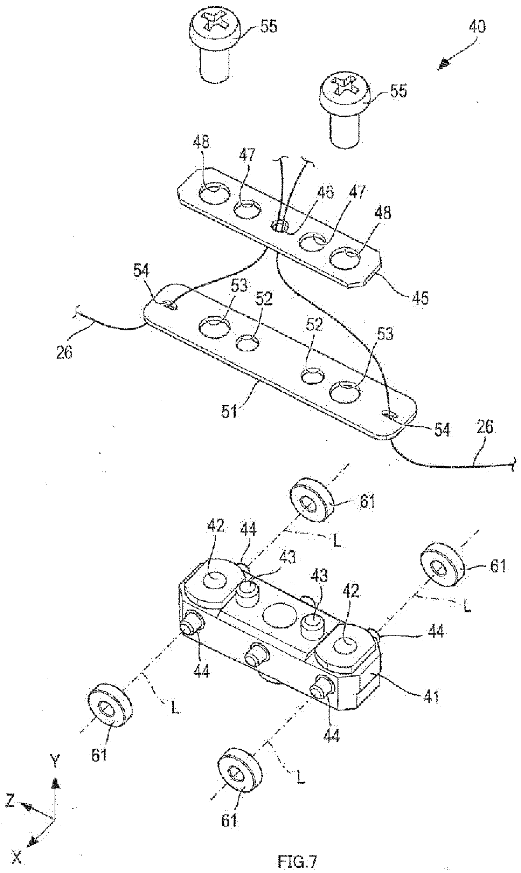

As shown in FIG. 7, the slider 40 is provided with a slider body 41, one first clamping portion 45, one second clamping portion 51, two fixing members 55, and four rolling members 61. The slider 40 may comprise an additional member.

The slider body 41 is a member formed in a pillar shape extending in the Z-axis direction. More specifically, the slider body 41 is a member formed in a square column shape. The slider body 41 is provided with two female screw holes 42, two positioning protrusions 43, and four rolling shafts 44.

The female screw holes 42 match male screws (described below) of the fixing members 55. The female screw holes 42 are screw holes used to hold the first clamping portion 45 and the second clamping portion 51. The two female screw holes 42 are screw holes formed to extend in the Y-axis direction, one of which is at an end part of the slider body 41 in the positive direction of the Z-axis, and the other of which is at an end part of the slider body 41 in the negative direction of the Z-axis.

The positioning protrusions 43 are protrusions that determine relative positions between the first clamping portion 45 and the second clamping portion 51 with respect to the slider body 41. The positioning protrusions 43 are members each having a cylindrical shape and protruding from the slider body 41 in the positive direction of the Y-axis. The two positioning protrusions 43 are provided at respective positions adjacent to the two female screw holes 42 and on a center part of the slider body 41 with respect to the two female screw holes 42.

The rolling shafts 44 are members that support the rolling members 61 in a rotatable manner about rotation axes L. The rolling shafts 44 are members each having a cylindrical shape and protruding from the slider body 41 in the X-axis direction. The center axis of each cylindrical shape is an axis parallel to the X-axis and is a corresponding one of the rotation axes L.

The four rolling shafts 44 are provided on the slider body 41, two of which are located, in the end part in the positive Z-axis direction, on a side face of the slider body 41 in the positive X-axis direction and a side face thereof in the negative X-axis direction, and the other two of which are located, in the end part in the negative Z-axis direction, on the side face of the slider body 41 in the positive X-axis direction and the side face thereof in the negative X-axis direction.

The first clamping portion 45 and the second clamping portion 51 are members that hold the wire 26 therebetween. The first clamping portion 45 and the second clamping portion 51 are overlapped and arranged on or above a surface of the slider body 41 located on a positive side in the Y-axis direction. The first clamping portion 45 is arranged on the second clamping portion 51 on the positive side in the Y-axis direction. In other words, the second clamping portion 51 is arranged on the first clamping portion 45 on the negative side in the Y-axis direction.

The first clamping portion 45 is configured to have a rectangular plate-like shape elongated in the Z-axis direction. The first clamping portion 45 includes one insertion hole 46, two first positioning holes 47, and two first fixing holes 48.

The insertion hole 46 is a through-hole through which an end of the wire 26 is inserted, and has a smaller diameter than the first positioning holes 47 and the first fixing holes 48 do. In the present embodiment, the insertion hole 46 is located in a central area of the first clamping portion 45 in the Z-axis direction.

The first positioning holes 47 are through-holes which are each formed to have an inner diameter larger than an outer diameter of each of the positioning protrusions 43 and through which the positioning protrusions 43 are inserted. In an attached state, the two first positioning holes 47 are formed in respective positions on the first clamping portion 45 that are opposite to the positioning protrusions 43. In the present embodiment, the two first positioning holes 47 are located in respective positions adjacent to the insertion hole 46, in other words, an adjacent position in the positive Z-axis direction and an adjacent position in the negative Z-axis direction.

The first fixing holes 48 are through-holes which are each formed to have an inner diameter larger than an outer diameter of each male screw of the fixing members 55 and through which the fixing members 55 are inserted. In the attached state, the first fixing holes 48 are formed in respective positions opposite to the female screw holes 42.

The two first fixing holes 48 are formed in respective positions on the first clamping portion 45 such that the two first positioning holes 47 are arranged between the first fixing holes 48. In the present embodiment, the two first fixing holes 48 are located, one of which is adjacent in the positive Z-axis direction to the first positioning hole 47 located on a positive side in the Z-axis direction, and the other of which is adjacent in the negative Z-axis direction to the first positioning hole 47 located on the negative side in the Z-axis direction.

The second clamping portion 51 is configured to have a rectangular plate-like shape elongated in the Z-axis direction. The second clamping portion 51 is configured to have a longer dimension in the Z-axis direction than the first clamping portion 45 has. The second clamping portion 51 includes two second positioning holes 52, two second fixing holes 53, and two guide holes 54.

The second positioning holes 52 are through-holes which are each formed to have an inner diameter larger than the outer diameter of each of the positioning protrusions 43 and through which the positioning protrusions 43 are inserted. The inner diameter of each second positioning hole 52 may be the same as or different from the inner diameter of each first positioning hole 47. In the attached state, the two second positioning holes 52 are formed in respective positions on the second clamping portion 51 that are opposite to the positioning protrusions 43.

The second fixing holes 53 are through-holes which are each formed to have an inner diameter larger than the outer diameter of each male screw of the fixing members 55 and through which the fixing members 55 are inserted. The inner diameter of each second fixing hole 53 may be the same as or different from the inner diameter of each first fixing hole 48.

In the attached state, the two second fixing holes 53 are formed in respective positions opposite to the female screw holes 42. The two second fixing holes 53 are formed on the second clamping portion 51 such that the two second positioning holes 52 are located between the two second fixing holes 53.

In the present embodiment, the two second fixing holes 53 are located, one of which is adjacent in the positive Z-axis direction to the second positioning hole 52 located on the positive side in the Z-axis direction, and the other of which is adjacent in the negative Z-axis direction to the second positioning hole 52 located on the negative side in the Z-axis direction.

The guide holes 54 are through-holes through which the wire 26 is inserted and are elongated holes extending in the Z-axis direction. The two guide holes 54 are formed on the second clamping portion 51 such that the two second fixing holes 53 are located between the two guide holes 54.

In the present embodiment, the two guide holes 54 are located in respective positions, one of which is adjacent in the positive Z-axis direction to the second fixing holes 53 located on the positive side in the Z-axis direction, and the other of which is adjacent in the negative Z-axis direction to the second fixing holes 53 located on the negative side in the Z-axis direction.

The positions where the two guide holes 54 are provided are contained in an area where the second clamping portion 51 protrudes more than the first clamping portion 45 in the positive Z-axis direction and an area where the second clamping portion 51 protrudes more than the first clamping portion 45 in the negative Z-axis direction, in a state in which the first clamping portion 45 and the second clamping portion 51 are attached to the slider body 41.

The fixing members 55 are configured to fix the first clamping portion 45 and the second clamping portion 51 in such a manner that the first clamping portion 45 and the second clamping portion 51 are arranged on or above the slider body 41. In addition, the fixing members 55 are configured to fix the first clamping portion 45 and the second clamping portion 51 with the wire 26 being interposed therebetween. In the present embodiment, as an example, a case in which the fixing members 55 are configured to include male screws is described.

The rolling members 61 are members that are supported by the rolling shafts 44 in a rotatable manner about the respective rotation axes L. The rolling members 61 are each formed in a cylindrical or columnar shape and configured to have a hole in a center through which the corresponding rolling shaft 44 is inserted.

The four rolling members 61 are arranged on the slider body 41, two of which are located, in the end part in the positive Z-axis direction, on the side face of the slider body 41 in the positive X-axis direction and the side face thereof in the negative X-axis direction, and the other two of which are located, in the end part in the negative Z-axis direction, on the side face of the slider body 41 in the positive X-axis direction and the side face thereof in the negative X-axis direction.

Next, an insulation between the end portions 27 of the wires 26 and the conductor 21A in the surgical instrument 1 with the aforementioned configurations is described below.

As shown in FIG. 4, when the sliders 40 move relatively along the driven grooves 21B, the wires 26 fixed to the sliders 40 are pulled or pushed as the sliders 40 move. As the wires 26 are pulled or pushed, positions of the end portions 27 of the wires 26 are changed.

More specifically, as shown in FIG. 3, the end portions 27 of the wires 26 move along the placement portion 25A, approaching or going away from the conductor 21A. Even when the end portions 27 of the wires 26 approach the conductor 21A, the specified length L1 between the end portions 27 of the wires 26 and the conductor 21A is maintained because the insulator 60 is located. The non-covering area 62 is always present and interposed between the end portions 27 of the wires 26 and the conductor 21A, so that the end portions 27 and the conductor 21A cannot be closer than a distance L1. This allows to maintain the insulation between the end portions 27 of the wires 26 and the conductor 21A.

According to the surgical instrument 1 with the aforementioned configuration, the end portions 27 of the wires 26 are covered by the insulator 60. The insulator 60 includes the non-covering area 62 with none of the wires 26 arranged inside the insulator 60, so that it is easier to maintain the insulation even without increasing a distance between the end portions 27 of the wires 26 and the conductor 21A.

More specifically, this configuration makes it possible to reduce a size of the casing 20 inside which the wires 26 and the conductor 21A are housed, and to set a smaller distance between ports in robotic endoscopic surgery. As a result, it is possible to apply robotic surgery to various surgical procedures.

The non-covering area 62 of the insulator 60 has the specific length L1 greater than or equal to the spatial distance, so that it is easier to maintain the insulation between the end portions 27 of the wires 26 and the conductor 21A.

The plurality of end portions 27 of the plurality of wires 26 are brought together and covered by the insulator 60. By covering the plurality of end portions 27 of the plurality of wires 26 with the insulator 60 together, it is easier to maintain the insulation between such plurality of end portions 27 and the conductor 21A.

## Claims

1. A surgical instrument (1) to be mounted on a surgical robot, the surgical instrument (1) comprising:
a casing (20);
a shaft (10) extending from the casing (20);
a movable part provided at a leading end of the shaft (10);
at least one transmitter (40) arranged to be relatively movable with respect to the casing (20);
at least one wire (26) having at least one portion held in the at least one transmitter (40), the at least one wire (26) transmitting movement of the at least one transmitter (40) to the movable part;
a conductor (21A) arranged in the casing (20), the conductor (21A) conducting an electric power that is externally supplied to the movable part;
**characterized by**
an insulator (60) having electrically insulating capacity, the insulator (60) covering an end portion (27) of the at least one wire (26) extending from the at least one transmitter (40) to a side and not connected to the movable part,
the insulator (60) being a tubular member made of an electrically insulating material, and the insulator (60) including:
a covering area (61) with the at least one wire (26) arranged inside the tubular member; and
a non-covering area (62) with none of the at least one wire (26) arranged inside the tubular member, and
the covering area (61) and the non-covering area (62) being arranged in a longitudinal direction of the insulator (60).

2. The surgical instrument (1) according to claim 1,
wherein the at least one transmitter (40) is arranged to be relatively movable in a linear manner with respect to the casing (20).

3. The surgical instrument (1) according to claim 1 or 2,
wherein the non-covering area (62) of the insulator (60) has a specified length greater than or equal to a spatial distance that is required to ensure electrical insulation and that is determined based on the electric power to be supplied to the conductor (21A), and
wherein the specified length is 3 mm or more and 80 mm or less.

4. The surgical instrument (1) according to any one of claims 1 to 3,
wherein the at least one wire (26) comprises a plurality of wires (26) and the end portion (27) comprises a plurality of end portions (27), and the insulator (60) covers a circumference of the plurality of end portions (27) of the plurality of wires (26).

## Patentansprüche

1. Chirurgisches Instrument (1) zum Montieren an einen Operationsroboter, wobei das chirurgische Instrument (1) aufweist:
ein Gehäuse (20);
einen Schaft (10), der sich vom Gehäuse (20) erstreckt;
ein bewegliches Teil, das an einem vorderen Ende des Schafts (10) bereitgestellt ist;
mindestens einen Überträger (40), der angeordnet ist, um in Bezug auf das Gehäuse (20) relativ beweglich zu sein;
mindestens einen Draht (26) mit mindestens einem Abschnitt, der in dem mindestens einen Überträger (40) gehalten wird, wobei der mindestens eine Draht (26) die Bewegung des mindestens einen Überträgers (40) zum beweglichen Teil überträgt;
einen Leiter (21A), der im Gehäuse (20) angeordnet ist, wobei der Leiter (21A) eine elektrische Energie leitet, die extern an das bewegliche Teil zugeführt wird; **gekennzeichnet durch**
einen Isolator (60) mit elektrischem Isolationsvermögen, wobei der Isolator (60) einen Endabschnitt (27) des mindestens einen Drahtes (26) abdeckt, der sich vom mindestens einen Überträger (40) auf eine Seite erstreckt und nicht mit dem beweglichen Teil verbunden ist,
wobei der Isolator (60) ein rohrförmiges Element ist, das aus einem elektrischen isolierenden Material gebildet ist, und der Isolator (60) einschließt:
eine abdeckende Fläche (61), bei der der mindestens eine Draht (26) im Inneren des rohrförmigen Elements angeordnet ist; und
eine nichtabdeckende Fläche (62), bei der keiner des mindestens einen Drahts (26) im rohrförmigen Element angeordnet ist, und
wobei die abdeckende Fläche (61) und die nichtabdeckende Fläche (62) in einer Längsrichtung des Isolators (60) angeordnet sind.

2. Chirurgisches Instrument (1) gemäß Anspruch 1,
wobei der mindestens eine Überträger (40) angeordnet ist, um in einer linearen Weise in Bezug auf das Gehäuse (20) relativ beweglich zu sein.

3. Chirurgisches Instrument (1) gemäß Anspruch 1 oder 2,
wobei die nichtabdeckende Fläche (62) des Isolators (60) eine spezifizierte Länge größer als oder gleich zu einem räumlichen Abstand aufweist, der erforderlich ist, um eine elektrische Isolierung sicherzustellen und der basierend auf der elektrischen Leistung bestimmt ist, die dem Leiter (21A) zuzuführen ist, und
wobei die spezifizierte Länge 3 mm oder mehr und 80 mm oder weniger beträgt.

4. Chirurgisches Instrument (1) gemäß einem der Ansprüche 1 bis 3,
wobei der mindestens ein Draht (26) eine Vielzahl von Drähten (26) aufweist und der Endabschnitt (27) eine Vielzahl von Endabschnitten (27) aufweist und der Isolator (60) einen Umfang der Vielzahl von Endabschnitten (27) der Vielzahl von Drähten (26) abdeckt.

## Revendications

1. Un instrument chirurgical (1) à monter sur un robot chirurgical, l'instrument chirurgical (1) comprenant :
un boîtier (20) ;
un arbre (10) s'étendant à partir du boîtier (20) ;
une partie mobile prévue à une extrémité avant de l'arbre (10) ;
au moins un transmetteur (40) disposé pour être relativement mobile par rapport au boîtier (20) ;
au moins un fil (26) ayant au moins une portion retenue dans l'au moins un transmetteur (40), l'au moins un fil (26) transmettant le mouvement de l'au moins un transmetteur (40) vers la partie mobile ;
un conducteur (21A) disposé dans le boîtier (20), le conducteur (21A) conduisant une alimentation électrique alimentée extérieurement à la partie mobile ; **caractérisé par**
un isolant (60) ayant une capacité d'isolation électrique, l'isolateur (60) couvrant une portion terminale (27) de l'au moins un fil (26) s'étendant de l'au moins un transmetteur (40) vers un côté et n'étant pas connecté à la partie mobile,
l'isolant (60) étant un élément tubulaire en matériau isolant électriquement, et
l'isolant (60) incluant :
une zone couvrante (61) avec au moins un fil (26) disposé à l'intérieur d'élément tubulaire ; et
une zone non couvrante (62) sans aucun des au moins un fil (26) disposés à l'intérieur d'élément tubulaire, et
la zone couvrante (61) et la zone non couvrante (62) étant disposées dans une direction longitudinale de l'isolant (60).

2. L'instrument chirurgical (1) selon la revendication 1,
dans lequel l'au moins un transmetteur (40) est disposé de manière relativement mobile de manière linéaire par rapport au boîtier (20).

3. L'instrument chirurgical (1) selon la revendication 1 ou 2,
dans lequel la zone non couvrante (62) de l'isolant (60) a une longueur spécifiée supérieure ou égale à une distance spatiale requise pour assurer l'isolation électrique et qui est déterminée en fonction de la puissance électrique à fournir au conducteur (21A), et
dans lequel la longueur spécifiée est de 3 mm ou plus et 80 mm ou moins.

4. L'instrument chirurgical (1) selon l'une des revendications 1 à 3,
dans lequel l'au moins un fil (26) comprend une pluralité de fils (26) et la portion terminale (27) comprend une pluralité de portions terminales (27), et l'isolant (60) couvre une circonférence de la pluralité des portions terminales (27) de la pluralité de fils (26).
